# EUROPEAN PATENT SPECIFICATION

(11) **EP 0 366 842 B1**
(45) Date of publication and mention of the grant of the patent: **13.10.1993**
(21) Application number: 88310366.5
(22) Date of filing: 03.11.1988
(51) Int. Cl.: C07C 33/22, C07C 29/88, C07C 29/80

(54) **Purification of phenyl ethyl alcohol**
Reinigung von Phenyläthylalkohol
Purification de l'alcool phényléthylique

(43) Date of publication of application: 09.05.1990
(73) Proprietor: BUSH BOAKE ALLEN Limited, London E17 5QP (GB)
(72) Inventor: Watts, Mark, Muswell Hill London NW10 2RL (GB)
(74) Representative: Lawrence, Peter Robin Broughton

(56) References cited:
- DD-A- 112 114
- CHEMICAL ABSTRACTS, vol. 93, no. 5, August 1980, page 874, abstract no. 46093f, Columbus, Ohio, US; N.N. ZELENETSKII et al.: "Development of a method for production of high-quality phenylethyl alcohol"

## Description

Phenyl ethyl alcohol (PEA, 2-phenyl ethanol) is widely used in flavours and perfumery products and for such purposes it must be substantially free of components that would impart an unsatisfactory odour to it.

PEA can be made commerically by syntheses that produce it in substantially pure form or in a form that is contaminated by impurities such as chloride. Methods are known for removing chloride contamination so as to provide a perfumery grade of PEA. One method that has been proposed in the literature is described in Chemical Abstracts 84 73865e and 93 46093. In this, chloride impurity is removed by reacting the impure product with an alkali metal or alkaline earth metal compound and distilling the PEA off from the metal chloride residue. The chloride-contaminated product typically will have been made by reaction of benzene and ethylene oxide in the presence of aluminium chloride.

There are, however, sources of PEA that are made by an entirely different process and that are free of chloride but are contaminated with phenolic and other impurities. The products contaminated with chloride would have been free of phenolic impurities as these would not have been by-products of the syntheses used for making the chloride-contaminated products. The presence of even trace quantities of phenols results in the product having a characteristic phenolic odour and thus renders it wholly unsuitable for perfumery uses. This is particularly unfortunate as a grade of crude PEA contaminated with phenolic impurity can be obtained very readily and cheaply as a by-product from various syntheses, including the synthesis of styrene.

Although methods are known that should permit the removal of phenolic impurities from PEA, in practice none of them are satisfactory. For instance the complexing method described in U.S. 4,400,558 is very expensive if performed under conditions to achieve adequate removal of phenolic impurities. Ion exchange might be usable but is expensive in equipment and materials. Solvent partitioning tends to be ineffective unless very large losses of PEA can be tolerated, and this renders it expensive. Probably the most obvious method, namely by washing with aqueous sodium hydroxide, is inadequately effective and/or involves high losses of PEA. This is because some of the sodium phenoxide that is formed during the washing or the phenol goes into the organic phase containing PEA and this organic phase has to be washed repeatedly to try to minimise the amount of phenol in the organic phase and this washing results in losses of PEA and is, in any event, generally inadequate for reducing the phenolic content sufficiently.

Particular problems are caused by the known sensitivity of PEA to undesired reactions. Thus in Chemical Abstracts Volume 27 page 1624 it is stated that PEA reacts at 250°C with alkaline dehydrating agent to form styrene. Potassium hydroxide, sodium hydroxide and barium oxide are mentioned. Also we believe that the optimum balance of odour properties in PEA may depend in part on it being contaminated with trace amounts of non-phenolic impurities and rigorous chemical treatment of the PEA may alter the odour properties undesirably.

Crude PEA is purified by azeotropic distillation using certain alkanolamines in a process described in EP 0185249. Although this technique may remove some impurities in a satisfactory manner it does not result in effective removal of phenolic impurities.

Accordingly, at present PEA contaminated with phenolic material is available as a very economic source of PEA, but it cannot economically be used for making perfumery grade material.

In the invention a purified PEA substantially free of phenolic odours is made from a crude PEA product that contains phenolic impurity by a process comprising
(i) forming a dry mixture comprising below 0.05% by weight water and the PEA contaminated with phenolic impurity that is substantially entirely in the form of a metal phenolate by a process comprising mixing the crude product with a metal or metal compound that will react with at least 99.95% by weight of the phenolic impurity to form the metal phenolate and
(ii) distilling purified PEA substantially free of phenolic odours from a residue comprising metal phenolate by distillation of the dry mixture under reduced pressure and collecting the distilled PEA.

If PEA is collected by distillation of a mixture that still contains water and/or unreacted phenolic impurity the distilled PEA will be contaminated with phenolic impurity to an undesirable, and generally unacceptable, extent. Thus if the mixture that is being distilled during the collection of purified PEA contains unreacted phenolic material, this material is liable to be distilled with the PEA. If the mixture contains water this is liable to hydrolyse the metal phenolate during the distillation so as to form free phenolic impurity which again will distill over with the PEA.

Accordingly it is preferred that distilled PEA is only collected when the mixture that is being distilled is free of water. If water is present at the start of distillation of PEA, the early distillate may be recycled or run to waste until all the water has been taken off. Preferably the reaction by which the phenolate is formed is forced to completion and the water is taken off before any PEA is taken off by distillation. For instance the PEA may be refluxed during the early stages or they may be completed under conditions that cause little or no distillation of PEA.

The crude PEA product generally contains at least 0.1% and in practice usually at least 0.5% of phenolic impurity and the amount can be up to, for instance, 5% or even 10% or higher. Generally however it is in the range of about 0.5 to 3%, typically around 1 to 2%.

The phenolic impurity may be any phenolic compound having undesirable odour properties and may therefore be, for instance, unsubstituted phenol or a methyl phenol but particular problems generally arise when the phenolic impurity comprises, and generally consists wholly or mainly of, ethyl phenol, often a mixture of ortho, meta and para ethyl phenols.

The PEA obtainable by the invention has a phenolic impurity level that is so low that the product appears substantially free of phenolic odours. In practice this generally requires that the phenolic impurity level in the purified PEA is below 0.05% (500 ppm) and generally below 0.02%. By the invention it is easily to obtain values of 0.01% (100 ppm) or lower. Although total freedom from phenolic impurity is of course desirable in practice the purified material generally contains at least 0.001% phenolic impurity as it may be uneconomic to aim for impurity levels below this.

The crude PEA product is normally a concentrated by-product obtained from styrene manufacture but can be any product in which PEA is contaminated by phenolic impurities. Preferably the purified PEA obtainable in the invention contains no other unwanted impurities and so is ready for perfumery or flavour use and so either the phenolic impurity is the only unwanted impurity in the crude starting product or any other impurities in it are removed during the process of the invention. For instance the crude PEA product may have been obtained by subjecting a by-product from styrene manufacture to other purification techniques before the process of the invention. Alternatively, the product obtainable in the process of the invention may be contaminated with other impurities and these may be removed subsquently by known techniques.

In order to form the metal phenolate, the crude PEA product is reacted with any metal or metal compound that under the conditions prevailing in the process will react with the phenolic impurity to form the metal phenolate and that will not cause degradation of PEA. Generally an alkali metal or alkaline earth metal, or a compound of such a metal, is used. Alkali metals that may be used as metals include sodium, lithium and potassium. Metal compounds that may be used are normally bases that have pKb greater than pKb of the phenolate that is being formed, but preferably very strong bases are avoided because of the risk that they will degrade the PEA. The compounds are generally formed of alkali metals, such as lithium, sodium or potassium, or alkaline earth metals, such as magnesium or calcium. The compounds may be hydroxides, hydrides, amides (substituted or unsubstituted) or alkoxides (substituted or unsubstituted) of such metals. The substituents in amides are generally hydrocarbon groups, typically C1-8, preferably C1-4, alkyl groups and the amides may be mono or di substituted. The alkoxides are typically formed of alkyl groups having 1 to 8 carbon atoms, preferably 1 to 4 carbon atoms, but the alkyl group may be substituted by, for instance, phenyl or amine. Suitable compounds that may be used in the invention include NaOH, KOH, LiOH, CaOH, MgOH, LiH, NaH, NaOEt, LiNH₂, LiNPr₂, NaOCH₂CH₂Ph and KOCH₂CH₂NH₂. A mixture of two or more different compounds may be used. The compound may be introduced as aqueous solution but the use of minimum or zero water is preferred as it minimises the amount of water that subsequently has to be distilled off.

The amount of metal or metal compound must be sufficient to convert all the phenolic impurity to metal phenolate and usually an excess is used. This excess may react with the PEA but provided most of the PEA, e.g., at least 80% and preferably at least 90%, remains in unreacted form this is acceptable. When a metal PEA derivative is formed it will remain with the metal phenolate at the end of the process and it is generally desirable to recover it for further processing. Unless a metal PEA is desired, the excess is usually 5 to 50% over stoichiometric.

One preferred process according to the invention deliberately uses metal PEA as part or all of the metal compound since this will react with the phenolic impurity to form free PEA and metal phenolate. A preferred process of this type is a cyclic process in which the substantially dry product comprises free PEA, metal phenolate and metal PEA derivative, the free PEA is removed by distillation under reduced pressure and part or all of the residue is then used as metal compound for reaction with fresh crude PEA. This cyclic process may be initiated by, for instance, reacting excess sodium hydroxide with crude PEA and then a proportion, typically from 25 to 75%, of the residue is recycled for use as the metal compound, usually in combination with additional metal or metal compound.

Another preferred process involves azeotropic distillation of hydrocarbon impurities using alkanolamine, as described in EP 0185249, followed by removal of purified PEA by the process of the invention. Conveniently the alkanolamine and the metal or metal compound are added before the azeotropic distillation. For instance the alkanolamine can be added as the potassium or other suitable derivative or the alkanolamine may be added together with another metal compound. For instance the crude PEA Product containing hydrocarbon and phenolic impurities may be blended with potassium hydroxide or other suitable alkali or alkaline earth metal or metal compound in an amount sufficient to convert all the phenolic impurities to metal phenolate and with ethanolamine or other alkanolamine in an amount and of a type as described in EP 0185249. For instance the materials added to the crude product may be potassium hydroxide in a 10% excess based on the stoichiometric amount of phenolic impurity and ethanolamine in an amount of 30% based on the weight of crude product. This mixture is then azeotroped to remove the ethanolamine or other alkanolamine and hydrocarbon impurities and subsequently distilled under reduced pressure to remove the remaining traces of water, and then under reduced pressure to remove the pure PEA.

Some reaction between the metal or metal compound and the phenolic impurities will often occur spontaneously upon admixture but it is desirable to force the reaction to substantial completion and to remove substantially all the water in order to ensure that the purified PEA is distilled from a product that is substantially free of unreacted phenolic impurity. If water is present hydrolysis of the metal phenolate, to form unreacted phenolic impurity, may occur during distillation. Very low residues of water and unreacted phenolic material may of course be tolerated provided their amounts are so low that they do not result in the purified PEA containing sufficient phenolic material to impart a phenolic odour to it. The amounts of unreacted phenolic material and water in the dry mixture are both below 0.05%, generally below 0.02% and most preferably below 0.01%.

The distillation must be at a temperature and pressure that does not cause substantial degradation of the PEA. Preferably the temperature is kept below 180°C, preferably not more than 160°C with maximum temperatures of 90 to 130°C often being preferred. The pressure is generally below 100 mbar and usually below 50 mbar. Generally it is above 10 mbar and usually above 20 or 30 mbar.

After the dried product has been formed, the distillation conditions are changed so as to take off the PEA. The PEA may have been refluxing during the initial reduced pressure distillation in which event removal of the purified PEA could be achieved by continuing the distillation under the same conditions but by taking the PEA off from the reflux column. Preferably however the distillation conditions are changed so as to promote distillation of PEA and, in particular, generally the distillation is conducted as a lower pressure, generally below 40 mbar and usually below 20 mbar. It is usually above 2, and generally above 5, mbar. The pressure typically is 10 to 50% of the pressure during the drying stage. The temperature should preferably be maintained below 165°C. The final temperature is usually above 100°C, often 140 to 160°C.

The following are examples.

### Example 1

Crude phenyl ethyl alcohol (1100g) containing 1-2% ethylphenols (a mixture of ortho, meta and para isomers) is charged to a distillation pot, containing 62g of a 46% solution of caustic soda in water. Water is distilled out at a pressure of 40 mbar to a final base temperature of 100°C. The pot contents are cooled and the pressure is reduced to 10 mbar.

Purified phenyl ethyl alcohol (1001g), containing less than 80 ppm total ethyl phenols, is collected as a distillate by gradually increasing the pot temperature to a final 160°C. The product has no phenolic odour and is suitable for perfumery use.

### Example 2

To the undistilled residue from example 1 (121g containing sodium ethyl phenates and sodium phenyl ethyl alkoxide) is charged crude phenyl ethyl alcohol (1100g containing 1-2% ethyl phenols). Any water that may, for example, have been dissolved in the phenyl ethyl alcohol, is distilled out at a pressure of 40 mbar to a final base temperature of 100°C. The pot contents are cooled and the pressure is reduced to 10 mbar.

Purified phenyl ethyl alcohol (1090g), containing less than 80ppm total ethylphenols, is then collected as a distillate by gradually increasing the pot temperature to a final 160°C.

## Claims

1. A process in which purified phenyl ethyl alcohol (PEA) substantially free of phenolic odours is made from a crude PEA product that contains phenolic impurity by a process comprising
(i) forming a dry mixture comprising below 0.05% by weight water and the PEA contaminated with phenolic impurity that is substantially entirely in the form of a metal phenolate by a process comprising mixing the crude product with a metal or metal compound that will react with at least 99.95% by weight of the phenolic impurity to form the metal phenolate and
(ii) distilling purified PEA substantially free of phenolic odours from a residue comprising metal phenolate by distillation of the dry mixture under reduced pressure and collecting the distilled PEA.

2. A process according to claim 1 in which the purified PEA that is distilled is collected only when the mixture that is being distilled is free of water.

3. A process according to claim 2 in which step (i) is forced to completion and all the water is taken off to produce a dry mixture before distilling PEA from the mixture.

4. A process according to any preceding claim in which the crude PEA product contains at least 0.5% phenolic impurity.

5. A process according to any preceding claim in which the phenolic impurity comprises ethyl phenol.

6. A process according to any preceding claim in which the purified PEA contains below 0.02% phenolic impurity.

7. A process according to any preceding claim in which the purified PEA contains 0.001% to 0.01% phenolic impurity.

8. A process according to any preceding claim conducted as a cyclic process in which the substantially dry product comprises free PEA, metal phenolate and metal PEA derivative, the free PEA is removed by distillation under reduced pressure and part or all of the residue is then used as metal compound for reaction with fresh crude PEA.

9. A process according to any preceding claim in which the crude PEA product also contains hydrocarbon impurities and a mixture is formed of this crude product with the metal or metal compound and with an alkanolamine and the mixture is then azeotroped to remove the alkanolamine and hydrocarbon impurities and is subsequently distilled under reduced pressure to remove remaining traces of water, and purified PEA is then distilled from the mixture.

10. A process according to any preceding claim in which the said metal is sodium.

11. A process according to any preceding claim in which the distillation is conducted at a temperature of below 130°C at a pressure below 100m.bar.

## Patentansprüche

1. Verfahren, bei dem gereinigter Phenylethylalkohol (PEA), der im wesentlichen frei von phenolischen Gerüchen ist, aus einem rohen PEA-Produkt, das phenolische Verunreinigung enthält, nach einem Verfahren hergestellt wird, umfassend
(i) Herstellen einer trockenen Mischung, umfassend unter 0,05 Gewichtsprozent Wasser und den mit phenolischer Verunreinigung, welche praktisch vollständig in Form eines Metallphenolats vorliegt, verunreinigten PEA, nach einem Verfahren, umfassend das Mischen des rohen Produkts mit einem Metall oder einer Metallverbindung, die mit mindestens 99,95 Gewichtsprozent der phenolischen Verunreinigung unter Bildung eines Metallphenolats reagieren, und
(ii) Destillieren von gereinigtem PEA, der im wesentlichen frei ist von phenolischen Gerüchen, aus einem Metallphenolat umfassenden Rückstand durch Destillation der trockenen Mischung unter vermindertem Druck und Sammeln des destillierten PEA.

2. Verfahren nach Anspruch 1, worin der gereinigte PEA, der destilliert wird, nur dann gesammelt wird, wenn das der Destillation unterworfene Gemisch wasserfrei ist.

3. Verfahren nach Anspruch 2, worin Schritt (i) bis zur Vollständigkeit durchgeführt wird und alles Wasser abgezogen wird, um eine trockene Mischung zu erhalten, bevor PEA aus der Mischung abdestilliert wird.

4. Verfahren nach irgendeinem vorangehenden Anspruch, worin das rohe PEA-Produkt mindestens 0,5% phenolische Verunreinigung enthält.

5. Verfahren nach irgendeinem vorangehenden Anspruch, worin die phenolische Verunreinigung Ethylphenol umfaßt.

6. Verfahren nach irgendeinem vorangehenden Anspruch, worin der gereinigte PEA unter 0,02% phenolische Verunreinigung enthält.

7. Verfahren nach irgendeinem vorangehenden Anspruch, worin der gereinigte PEA 0,001 bis 0,01% phenolische Verunreinigung enthält.

8. Verfahren nach irgendeinem vorangehenden Anspruch, durchgeführt als Kreisprozess, bei dem das im wesentlichen trockene Produkt freien PEA, Metallphenolat und Metall-PEA-Derivat umfaßt, der freie PEA durch Destillation unter vermindertem Druck entfernt wird und ein Teil oder der gesamte Rückstand dann als Metallverbindung für die Reaktion mit frischem rohem PEA verwendet wird.

9. Verfahren nach irgendeinem vorangehenden Anspruch, worin das rohe PEA-Produkt auch Kohlenwasserstoff-Verunreinigungen enthält und eine Mischung aus diesem rohen Produkt mit dem Metall oder der Metallverbindung und mit einem Alkanolamin gebildet wird und die Mischung dann azeotrop destilliert wird, um das Alkanolamin und Kohlenwasserstoff-Verunreinigungen zu entfernen, und anschließend unter vermindertem Druck destilliert wird, um restliche Spuren von Wasser zu entfernen, und dann gereinigtes PEA aus der Mischung destilliert wird.

10. Verfahren nach irgendeinem vorangehenden Anspruch, worin das Metall Natrium ist.

11. Verfahren nach irgendeinem vorangehenden Anspruch, worin die Destillation bei einer Temperatur unter 130°C und einem Druck unter 100 mbar durchgeführt wird.

## Revendications

1. Procédé dans lequel on fabrique de l'alcool phényléthylique (PEA) purifié qui n'a pratiquement pas d'odeurs phénoliques à partir d'un PEA brut qui contient une impureté phénolique à l'aide d'un procédé comprenant :
(i) la formation d'un mélange sec comprenant moins de 0,05 % en poids d'eau et le PEA contaminé par une impureté phénolique qui est pratiquement totalement sous forme d'un phénolate de métal, à l'aide d'un procédé comprenant le mélange du produit brut avec un métal ou un composé métallique qui va réagir avec au moins 99,95 % en poids de l'impureté phénolique pour former le phénolate de métal et
(ii) la séparation par distillation du PEA purifié qui n'a pratiquement pas d'odeurs phénoliques d'un résidu comprenant le phénolate de métal, par distillation du mélange sec sous pression réduite, et récupération du PEA distillé.

2. Procédé selon la revendication 1, dans lequel on ne récupère le PEA purifié qui est distillé que quand le mélange que l'on distille ne contient plus d'eau.

3. Procédé selon la revendication 2, dans lequel on provoque l'achèvement de l'étape (i) et on élimine toute l'eau afin de produire un mélange sec avant de séparer le PEA du mélange par distillation.

4. Procédé selon l'une quelconque des revendications précédentes, dans lequel le produit constitué de PEA brut contient au moins 0,5 % d'impureté phénolique.

5. Procédé selon l'une quelconque des revendications précédentes, dans lequel l'impureté phénolique comprend l'éthylphénol.

6. Procédé selon l'une quelconque des revendications précédentes, dans lequel le PEA purifié comprend moins de 0,02 % d'impureté phénolique.

7. Procédé selon l'une quelconque des revendications précédentes, dans lequel le PEA purifié contient de 0,001 à 0,01 % d'impureté phénolique.

8. Procédé selon l'une quelconque des revendications précédentes mis en oeuvre en tant que procédé cyclique, dans lequel le produit pratiquement sec contient du PEA libre, du phénolate de métal et un dérivé métallique du PEA, le PEA libre est éliminé par distillation sous pression réduite, et une partie du résidu ou tout le résidu est utilisé en tant que composé métallique destiné à réagir avec du PEA brut frais.

9. Procédé selon l'une quelconque des revendications précédentes, dans lequel le produit constitué de PEA brut contient aussi des impuretés constituées d'hydrocarbures et dans lequel on forme un mélange de ce produit brut, du métal ou du composé métallique et d'une alcanolamine, et on sépare ensuite le mélange avec azéotrope pour éliminer l'alcanolamine et les impuretés constituées d'hydrocarbures, et on distille ensuite sous pression réduite pour éliminer les traces d'eau restantes, et ensuite on sépare le PEA purifié du mélange par distillation.

10. Procédé selon l'une quelconque des revendications précédentes, dans lequel ledit métal est du sodium.

11. Procédé selon l'une quelconque des revendications précédentes, dans lequel la distillation est mise en oeuvre à une température inférieure à 130°C et à une pression inférieure à 100 mbar.
